# FASCICULE DE BREVET EUROPEEN

(11) **EP 0 775 217 B1**
(45) Date de publication et mention de la délivrance du brevet: **06.05.1998**
(21) Numéro de dépôt: 95927752.6
(22) Date de dépôt: 03.08.1995
(51) Int. Cl.: C12Q 1/68

(54) **Méthode d'amplification et/ou de détection d'une séquence d'acide nucléique, réactif de détection et leurs applications**
Verfahren zur Amplifizierung und/oder Nachweis einer Nukleinsäuresequenz, Nachweisreagenz und deren Verwendungen
Method for the amplification and/or detection of a nucleic acid sequence, detection reagent and uses thereof

(30) Priorité: 10.08.1994 FR 9409896
(43) Date de publication de la demande: 28.05.1997
(73) Titulaire: CIS BIO INTERNATIONAL, 91400 Saclay (FR)
(72) Inventeur: SAUVAIGO, Sylvie, F-38320 Herbeys (FR); BAZIN, Hervé, F-38000 Grenoble (FR); LIVACHE, Thierry, F-38000 Grenoble (FR)
(74) Mandataire: Ores, Irène
(86) Numéro de dépôt international: FR9501047
(87) Numéro de publication internationale: WO9605323

(56) Documents cités:
- EP-A- 0 427 074
- EP-A- 0 545 010
- WO-A-89/09285
- WO-A-90/02819
- WO-A-92/12261
- FR-A- 2 697 851

## Description

La présente Invention est relative à une méthode d'amplification et/ou de détection d'une séquence spécifique d'acide nucléique (séquence cible), mettant en oeuvre une étape d'hybridation, à un réactif approprié à ladite amplification et/ou détection ainsi qu'à leurs applications dans le domaine du diagnostic médical et vétérinaire ainsi que dans l'agro-alimentaire pour l'amplification, la détection et la quantification de séquences d'acide nucléique.

La détection et/ou la quantification de séquences d'acide nucléiques spécifiques (séquences cibles) est une technique de plus en plus utilisée pour l'identification et la classification de microorganismes et le diagnostic des maladies infectieuses.

La technique de base pour réaliser une telle détection est la méthode d'hybridation simple : cette méthode est basée sur la capacité d'association de deux brins d'acide nucléique qui contiennent des séquences complémentaires, pour former une séquence double-brin. Une séquence cible peut ainsi s'hybrider avec une séquence dénommée sonde. Le marquage préalable de la sonde ou de l'acide nucléique cible permet, après séparation de l'hybride et de la sonde résiduelle simple-brin, la détection du duplex formé. Cette méthode, relativement simple à mettre en oeuvre présente, toutefois, un certain nombre d'inconvénients : elle manque généralement de sensibilité pour les applications en diagnostic médical ; de plus, les méthodes de détection de l'hybride formé font appel à des techniques qui ne sont pas compatibles avec une utilisation en routine de ces essais.

Pour pallier ce manque de sensibilité des tests d'hybridation et essayer de conserver une bonne spécificité du test, de nombreuses améliorations de cette technique de base ont été développées et concernent :
- soit l'automatisation du test : faciliter la séparation des duplex séquence cible-sonde formés par couplage par hybridation à un support solide,
- soit essentiellement la sensibilité : amplifier de façon spécifique les séquences cibles, de manière à rendre la détection effectivement plus sensible. Plusieurs méthodes ont été proposées pour amplifier les séquences cibles :

. la réaction de polymérisation en chaîne *(Polymerase Chain Reaction* ou PCR) décrite par MULLIS et al. (Brevets US 4,683,195, 4,683,202, 4,800,159, EP 86302298.4, 86302299.2 et 87300203.4) est un processus cyclique à trois températures, basé sur l'extension par une ADN polymérase de deux amorces spécifiques hybridées sur la séquence cible et qui permet l'amplification exponentielle de la séquence recherchée. Ce système nécessite la répétition séquentielle des séquences suivantes : dénaturation de l'ADN cible, hybridation des amorces sur les brins complémentaires de la séquence cible et élongation par une ADN polymérase des extrémités 3' des amorces en regard. Une polymérase thermostable est généralement utilisée et le processus se déroule de façon entièrement automatique dans des appareils spécifiques. Cette technique a l'inconvénient majeure d'être trop sensible et d'entraîner des résultats faussement positifs.
. une variante de la PCR, connue sous le nom de *Ligase Chain Reaction* (LCR) (Brevet EP 0 320 308), qui nécessite 4 sondes oligonucléotidiques, est basée sur la ligation par une ligase de deux de ces sondes oligonucléotidiques, s'hybridant conjointement sur la même séquence cible.

Ces deux sondes oligonucléotidiques ont la particularité d'être contigües sur le brin cible, par l'extrémité 3' de l'un et l'extrémité 5' de l'autre.
. les propriétés des ARN polymérase ADN-dépendantes extraites de bactériophages, sont également utilisées dans des systèmes d'amplification, et permettent l'obtention de nombreuses copies de transcrits. Par exemple, l'amplification PCR a été couplée à l'amplification par transcription grâce à l'incorporation de séquences promoteur d'ARN polymérase dans l'une ou dans les deux amorces d'amplification. L'ADN double-brin amplifié sert de substrat pour la transcription d'ARN simple-brin (MURAKAWA et al., DNA, 1988, 7, 287-295) ou double-brin (Demande Internationale PCT WO 93/12229, au nom de la Demanderesse).

Ceci est également le cas dans une méthode d'amplification, basée sur la transcription, dénommée la TAS *(Transcription Amplification System)* (Demande Internationale PCT WO 88/10315). Dans cette méthode, des amorces contenant le promoteur d'une ARN polymérase sont hybridées avec une séquence cible et leur extension est réalisée à l'aide d'une ADN polymérase. L'ADN double-brin résultant est transcrit en ARN qui peut lui-même servir de matrice pour l'hybridation des amorces et donc la transcription de nouvelles séquences d'ARN.

D'autres méthodes basées sur la production de transcrits, pour amplifier une séquence à détecter, ont été également décrites
. la Demande de Brevet EP 0 587 266, au nom de GEN-PROBE INCORPORATED, décrit une méthode d'amplification d'acide nucléique, qui comprend :
   (i) l'incubation d'un mélange consistant essentiellement en la séquence nucléique à détecter (ARN ou ADN) avec un ou plusieurs oligonucléotides dits "promoteurs-amorces", comprenant une séquence suffisamment complémentaire de l'extrémité 3' de la séquence-cible à hybrider, servant d'amorce et un promoteur pour une ARN polymérase, et
   (ii) la formation d'un hybride promoteur-amorce/séquence cible.
. L'article paru dans Clin. Chem., (1993, 39, 9, 1934-1938) et les Demandes de Brevet EP 0 427 073 et EP 0 427 074, au nom de MOLECULAR DIAGNOSTICS, INC., décrivent une sonde d'ADN qui peut être amplifiée transcriptionellement, ladite sonde ayant une structure en épingle à cheveux, comprenant une séquence complémentaire d'une séquence cible à détecter et une séquence promoteur double-brin.
. Un autre système d'amplification est basé sur le couplage d'une sonde ARN à de l'ARN (Brevet US 4,786,600) qui sert de substrat à la Q-β-réplicase, permettant la synthèse de copies multiples de la sonde.

La Demande Internationale PCT WO 91/10746, au nom de CHIRON CORPORATION, décrit une amplification du signal après hybridation par l'intermédiaire de séquences promoteur d'ARN polymérase couplées à une sonde, qui entraîne la production de transcrits complémentaires d'une matrice associée au promoteur mais différente de la séquence cible. Toutefois, l'ensemble de ces améliorations, si elles conduisent à une augmentation de la sensibilité, ont pour conséquence une perte de spécificité.

Pour pallier cet inconvénient, la présente Invention s'est en conséquence donné pour but de pourvoir à un réactif et à une méthode aptes à améliorer de manière significative la sensibilité des tests d'hybridation, tout en conservant la spécificité de la reconnaissance de la séquence cible obtenue avec les sondes, ce qui répond mieux aux besoins de la pratique que les réactifs et méthodes de l'Art antérieur.

La présente invention a pour objet un procédé d'amplification d'au moins une séquence d'acide nucléique, caractérisé en ce qu'il comprend :
(1) l'hybridation en solution d'au moins une séquence cible avec une construction polynucléotidique comprenant au moins un domaine (A) simple-brin correspondant à une séquence nucléotidique complémentaire de la séquence cible et un domaine (B,B') double-brin constituant un promoteur,
(2) l'élimination des acides nucléiques non hybridés avec le domaine (A) de ladite construction polynucléotidique,
(3) la digestion par une nucléase spécifique des acides nucléiques simple-brin des fragments simple-brin des constructions polynucléotidiques, c'est-à-dire notamment celles n'ayant pas formé d'hybrides avec la séquence cible, et
(4) la transcription en solution et en présence d'une ARN-polymérase ADN-dépendante, de la séquence cible ayant hybridé à l'étape (1), sous la forme de copies multiples d'ARN de ladite séquence cible.
   On entend par séquence cible, au sens de la présente invention, aussi bien de l'ADN double-brin, de l'ADN simple-brin, de l'ARN double-brin que de l'ARN simple-brin.
   Selon un mode de mise en oeuvre avantageux dudit procédé d'amplification, l'étape (2) de séparation comprend avantageusement une étape de précipitation des acides nucléiques.
   Selon un autre mode de mise en oeuvre avantageux dudit procédé d'amplification, la construction polynucléotidique de l'étape (1) comprend au moins un domaine (A) simple-brin correspondant à une séquence nucléotidique complémentaire de la séquence cible, un domaine (B,B') double-brin constituant un promoteur, un domaine (C) constitué par un bras espaceur, non hydrolysable par des nucléases spécifiques des acides nucléiques simple-brin et un domaine (D) constitué par un système de fixation à un support.
   Les domaines (C) et (D) sont plus précisément définis ci-dessous.
   Conformément à ce mode de mise en oeuvre, l'étape (2) de séparation comprend avantageusement une étape de fixation sur un support approprié des constructions polynucléotidiques comportant le domaine (D), suivie d'une étape d'élimination des éléments non fixés.
   Selon une disposition avantageuse de ce mode de mise en oeuvre, la capacité du support est choisie de telle sorte que tous les composés comportant le domaine (D) puissent se fixer, sans qu'il y ait compétition entre eux.
   De manière préférée, le support présente les caractéristiques suivantes :
   - il est choisi parmi les tubes, les microplaques et les microbilles,
   - il est recouvert d'une substance apte à fixer le domaine (D), telle que de l'avidine,
   - il présente une capacité de fixation supérieure ou égale à 8 pmoles de domaines (D), notamment constitué par de la biotine,
   - le rapport entre la capacité du support, en pmoles et le nombre de pmoles d'espèces présentes en solution et présentant une affinité avec ledit support est supérieure à 1.

   La présente invention a également pour objet un procédé de détection et/ou de quantification d'au moins une séquence d'acide nucléique, caractérisé en ce qu'il comprend, outre les étapes (1), (2), (3) et (4) précitées, à savoir : (1) l'hybridation en solution d'au moins une séquence cible avec une construction polynucléotidique comprenant au moins un domaine (A) simple-brin correspondant à une séquence nucléotidique complémentaire de la séquence cible et un domaine (B,B') double-brin constituant un promoteur, (2) l'élimination des acides nucléiques non hybridés avec le domaine (A) de ladite construction polynucléotidique, (3) la digestion par une nucléase spécifique des acides nucléiques simple-brin des fragments simple-brin des constructions polynucléotidiques, c'est-à-dire notamment celles n'ayant pas formé d'hybrides avec la séquence cible à détecter, et (4) la transcription en solution et en présence d'une ARN-polymérase ADN-dépendante, de la séquence cible ayant hybridé à l'étape (1), sous la forme de copies multiples d'ARN de ladite séquence cible,
(5) la détection et/ou la quantification des transcrits produits.

L'étape (4) de transcription et l'étape (5) de détection du transcrit par hybridation d'une sonde peuvent avoir lieu simultanément.

Selon un mode de mise en oeuvre avantageux dudit procédé de détection, l'étape (2) de séparation comprend avantageusement une étape de précipitation des acides nucléiques.

Selon un autre mode de mise en oeuvre avantageux dudit procédé de détection, la construction polynucléotidique de l'étape (1) comprend au moins un domaine (A) simple-brin correspondant à une séquence nucléotidique complémentaire de la séquence cible, un domaine (B,B') double-brin constituant un promoteur, un domaine (C) constitué par un bras espaceur, non hydrolysable par des nucléases spécifiques des acides nucléiques simple-brin et un domaine (D) constitué par un système de fixation à un support.

Conformément à ce mode de mise en oeuvre, l'étape (2) de séparation comprend avantageusement une étape de fixation sur un support approprié des constructions polynucléotidiques comportant le domaine (D), suivie d'une étape d'élimination des éléments non fixés.

Dans ce cas, l'étape (2) de fixation sur un support, après hybridation avec la séquence cible, des ensembles construction polynucléotidique/séquence cible permet d'éliminer les composés présents dans le milieu d'hybridation et d'effectuer facilement les lavages nécessaires après chaque étape du procédé.

De manière générale, l'étape (3) de digestion nucléasique, qui est essentielle aussi bien dans la mise en oeuvre du procédé d'amplification que du procédé de détection, permet d'éliminer les domaines (A) n'ayant pas réagi (absence d'hybridation), assurant ainsi une spécificité importante auxdits procédés, tout en ayant une sensibilité significativement améliorée, par rapport aux tests d'hybridation et/ou d'amplification classiques.

En effet, lorsque le domaine (A) (complémentaire de la séquence cible à amplifier ou à détecter) est digéré, il ne peut plus servir de matrice à la transcription et celle-ci n'a pas lieu. Certaines enzymes spécifiques des simples-brins vont laisser intacts des nucléotides courts (1 à 6 bases de long) simple-brin, après digestion ; toutefois, lorsque cela se produit, les transcrits obtenus sont de petite taille (maximum 6 nucléotides de long) et peuvent être aisément distingués des transcrits présentant la taille du domaine (A).

Lorsque le domaine (A) est hybridé de façon parfaite avec la séquence cible, il est protégé de l'action des nucléases spécifiques des simples-brins et peut servir de matrice pour la transcription.

Selon un autre mode de mise en oeuvre avantageux desdits procédés (amplification et détection), l'étape (1) comprend simultanément la formation in situ de la construction polynucléotidique par hybridation d'une séquence comprenant au moins de 5' en 3' le domaine (A) et le domaine (B) avec une séquence comprenant au moins le domaine (B') et de préférence en outre le domaine (D) et le domaine (C) et l'hybridation de ladite construction polynucléotidique ainsi formée et de la séquence cible.

Dans le cas où la construction polynucléotidique comprend les domaines (D) et (C), l'étape (2) de fixation sur un support, après hybridation avec la séquence cible, des ensembles construction polynucléotidique/séquence cible et séquences comprenant le domaine (D), le domaine (C) et le domaine (B') non appariées permet d'éliminer les composés présents dans le milieu d'hybridation et d'effectuer facilement les lavages nécessaires après chaque étape desdits procédés, et l'étape (3) permet la digestion des domaines (A) non appariés et des constructions incomplètes (domaine (B') non apparié au domaine (B)). Par contre, le bras espaceur n'est pas digéré.

En ce qui concerne plus précisément la mise en oeuvre du procédé de détection conforme à l'invention : l'étape (4) de transcription est réalisée en présence de désoxynucléotides modifiés tels que l'α-³²P-UTP, le dinitrophényl-UTP, la biotine-UTP, la digoxigénine-UTP. Ceci permet l'incorporation dans les transcrits, de marqueurs, pouvant servir à leur détection et à leur quantification, lors de l'étape (5) mise en oeuvre dans le procédé de détection.

Conformément à l'invention, lorsque les transcrits sont réalisés en présence de désoxynucléotides modifiés, l'étape (5) de détection comprend la capture desdits transcrits modifiés par une sonde spécifique d'une portion située au centre ou à l'extrémité 3' desdits transcrits et la détection des marqueurs incorporés dans lesdits transcrits par des systèmes de révélation convenables.

Selon ce dernier mode de mise en oeuvre du procédé de détection, la sonde de détection fait avantageusement entre 15 et 200 bases de long et peut être modifiée à une de ses extrémités par une molécule permettant sa fixation sur un support. Cette molécule de fixation est préférentiellement un haptène ou une biotine.

En variante, le transcrit et sa sonde de détection peuvent être hybridés en solution, dans des conditions favorisant la formation du duplex spécifique et les hybrides ainsi formés peuvent être immobilisés sur supports modifiés.

Selon encore une autre variante, l'immobilisation de la sonde sur le support peut être préalable à l'étape de détection (immobilisation sur microbilles par exemple et addition des microbilles dans le milieu de transcription).

Les transcrits non modifiés (non marqués) peuvent être également détectés après leur fixation sur support (dépôt par dot-blot sur membrane de nylon, par exemple), par hybridation avec une sonde de détection spécifique marquée, telle que définie ci-dessus.

Aussi bien le procédé d'amplification que le procédé de détection répondent au besoin exposé ci-dessus, à savoir ils sont à la fois sensibles et spécifiques ; en particulier, dans le cas de la mise en oeuvre d'une construction polynucléotidique comprenant un domaine (C) et un domaine (D), seule la matrice nécessaire à la transcription est fixée sur le support ; en conséquence, le milieu de transcription ne contient que les transcrits spécifiques de la séquence cible à détecter.

La présente invention a également pour objet une construction polynucléotidique apte à amplifier et/ou à détecter une séquence cible d'acide nucléique, caractérisée en ce qu'elle comprend :
(a) un premier domaine (A), qui est simple-brin et comprend une séquence nucléotidique complémentaire de la séquence cible,
(b) un deuxième domaine (B,B'), situé à l'extrémité 3' dudit domaine (A), qui est formé de deux séquences complémentaires (B) et (B') et forme un promoteur double-brin d'une ARN polymérase ADN-dépendante,
(c) un troisième domaine (C), constitué par un bras espaceur, non hydrolysable par des nucléases spécifiques des acides nucléiques simple-brin, et
(d) un quatrième domaine (D), constitué par un système de fixation du domaine (B,B') sur un support solide convenable,
lesquels domaines (C) et (D) peuvent être avantageusement fixés soit en 5' du domaine (B'), soit en 5' du domaine (A), soit en 3' du domaine (B).

Ladite construction polynucléotidique comprend donc un segment, également dénommé sonde ci-après, qui correspond soit aux domaines (D)+(C)+3'-(B)+(A)-5', soit aux domaines 3'-(B)+(A)-5'+(C)+(D), soit aux domaines 3'-(B)+(A)-5' et un segment, également dénommé promoteur ci-après, qui correspond soit aux domaines (D)+(C)+5'-(B')-3', soit au domaine (B'), selon le lieu d'accrochage des domaines (C) et (D).

Selon un mode de réalisation avantageux de ladite construction, le domaine (B,B') constitue l'un des promoteurs suivants : promoteur de la T7 ARN polymérase, promoteur de la T3 ARN polymérase, promoteur de la SP6 ARN polymérase ; également de manière avantageuse, des bases G ou C peuvent être ajoutées à l'extrémité 3' du domaine B, de façon à améliorer la cohésion de la partie double-brin de ladite construction. L'ajout de telles bases ne modifie toutefois pas les rendements ultérieurs de transcription.

Des séquences peuvent également être ajoutées à l'extrémité 5' dudit domaine B, avant la région correspondant à la séquence complémentaire de la séquence cible (domaine (A)).

Conformément à l'invention, la taille de la séquence formée par les domaines (A) et (B) est comprise entre 30 et 200 bases.

Selon un autre mode de réalisation avantageux de l'invention, le domaine (C) ou bras espaceur est avantageusement constitué de 2 à 10, de préférence de 4 à 6 synthons, choisis parmi les alkanes-diol ou les synthons décrits dans la Demande Internationale PCT/FR94/00354, au nom de la Demanderesse, qui répondent à l'une des formules suivantes :

-R₁-[(CH₂)ₙ-R₂]ₓ- [(CH₂)ₘ-R₃]_{y}- (CH₂)ₚ-

dans laquelle :
n est un nombre entier de 1 à 10 ;
m est égal à 0 ou est un nombre entier de 1 à 10 ;
p est égal à 0 ou est un nombre entier de 1 à 10 ;
x est égal à 0 ou est un nombre entier de 1 à 8 ;
y est égal à 0 ou est un nombre entier de 1 à 8 ;
R₁, R₂ et R₃, qui peuvent être identiques ou différents représentent
   CH₂ ; O ; S ; NR' ; CO ; CH=CH ; NR'CO ; CONR' ; NHSO₂ ; R'PO₄,
où R' représente un atome d'hydrogène ou une chaîne alkyle en C₁ à C₁₂.

De tels bras espaceurs éloignent le promoteur du support solide sur lequel il est susceptible de se fixer, afin de permettre effectivement la transcription en ARN, par l'accrochage de l'ARN polymérase sur le promoteur double-brin, en évitant ainsi tout problème d'encombrement stérique.

Ils peuvent avantageusement être obtenus par incorporation de synthons tels que définis ci-dessus pendant la synthèse oligonucléotidique du domaine (B'), comme décrit notamment dans WILK et al. (Nucleic Acids Res., 1990, **18**, 8, 2065-2068), tel que le synthon C-16-*spacer®* PA (Cambridge Research Biochemicals), à partir de dérivés phosphoramidites de squelettes carbonés, du type 1-O-diméthoxytrityl-butane-3-O-(2-cyanoéthyl-N,N-diisopropyl) phosphoramidite ou selon la méthode décrite dans la Demande Internationale PCT/FR94/00354 précitée.

Egalement conformément à l'invention, le domaine (D) est une molécule pouvant avoir une affinité pour une autre molécule immobilisable sur support solide ; avantageusement il s'agit d'un oligonucléotide, d'un haptène, du dinitrophényle, d'une biotine, ou d'une digoxigénine.

La construction polynucléotidique conforme à l'invention permet la synthèse de transcrits d'ARN, dont la séquence correspond à la séquence cible, en présence d'ARN polymérase-ADN dépendante. 10 à 10⁴ transcrits peuvent être obtenus à partir d'une seule molécule de séquence cible, permettant ainsi l'amplification de la séquence cible et ainsi un accroissement significatif de la sensibilité.

La présente invention a également pour objet un procédé de production d'une construction polynucléotidique conforme à l'invention, caractérisé en ce qu'il comprend :
(a) la synthèse du segment dénommé promoteur comprenant le domaine (D) (système d'accrochage), le domaine (C) (bras espaceur) et le domaine 5'-(B')-3' (correspondant à un simple-brin d'un promoteur),
(b) la synthèse du segment dénommé sonde, comprenant le domaine 3'-(B) (correspondant à une séquence simple-brin complémentaire de (B')), le domaine (A)-5' (séquence simple-brin complémentaire de la séquence cible à détecter) et
(c) l'hybridation des domaines (B) et (B'), des segments obtenus en (a) et (b), dans des conditions stringentes.

Conformément à ce procédé, l'étape (b) comprend la synthèse chimique dudit fragment, notamment à l'aide de la technologie des PAC-phosphoramidites.

En variante, l'étape (b) comprend une amplification PCR asymétrique d'une séquence cible témoin avec des amorces spécifiques de ladite séquence cible, l'une desdites amorces comportant la séquence (B) à son extrémité 5'.

En variante, ledit procédé de production d'une construction polynucléotidique conforme à l'invention, comprend :
(a) la synthèse du segment dénommé promoteur comprenant uniquement le domaine 5'-(B')-3' (correspondant à un simple-brin d'un promoteur),
(b) la synthèse du segment dénommé sonde, comprenant le domaine (B) (correspondant à une séquence simple-brin complémentaire de (B')), le domaine (A)-5' (séquence simple-brin complémentaire de la séquence cible à détecter) et en 3'ou en 5' dudit segment, le domaine (C) (bras espaceur) et le domaine (D) (système d'accrochage), et
(c) l'hybridation des domaines (B) et (B'), des segments obtenus en (a) et (b), dans des conditions stringentes.

Outre les dispositions qui précèdent, l'invention comprend encore d'autres dispositions, qui ressortiront de la description qui va suivre, qui se réfère à des exemples de mise en oeuvre du procédé objet de la présente invention, ainsi qu'aux dessins annexés dans lesquels :

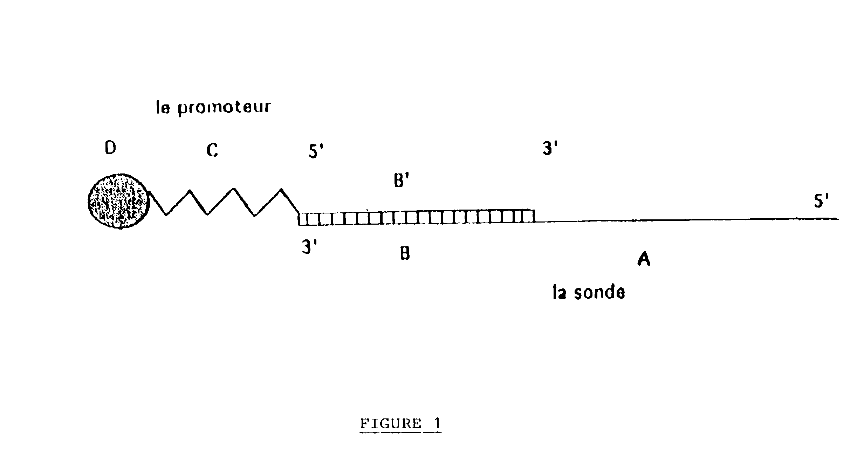

- la figure 1 représente une construction polynucléotidique conforme à l'invention.

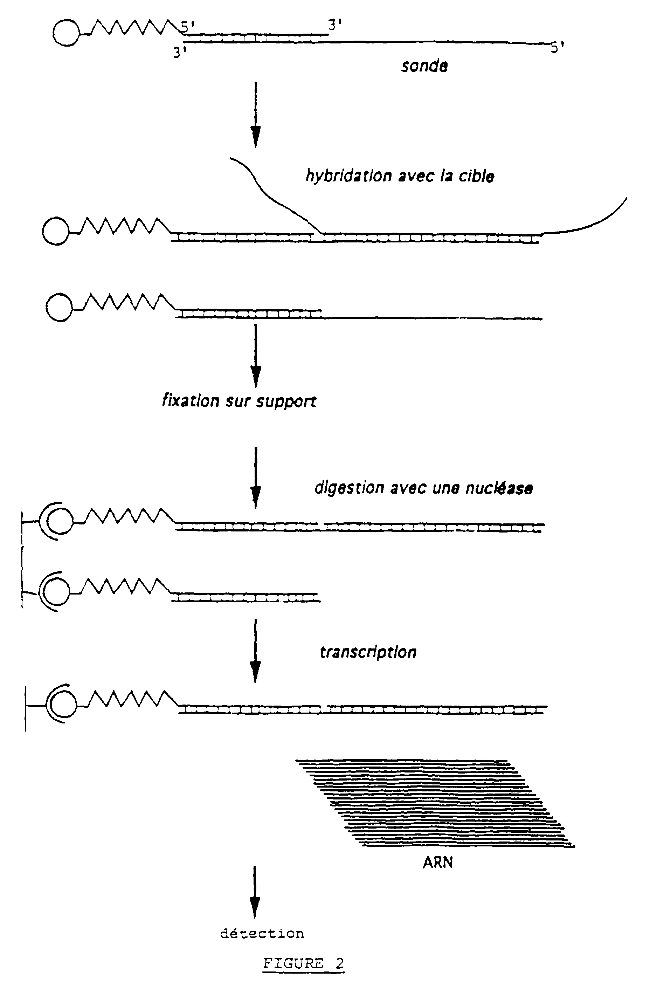

- la figure 2 illustre le procédé de détection (et/ou de quantification) d'au moins une séquence cible, conforme à l'invention.
- les figures 3, 4 et 6 illustrent l'augmentation significative de la sensibilité du test d'hybridation par le procédé conforme à l'invention.

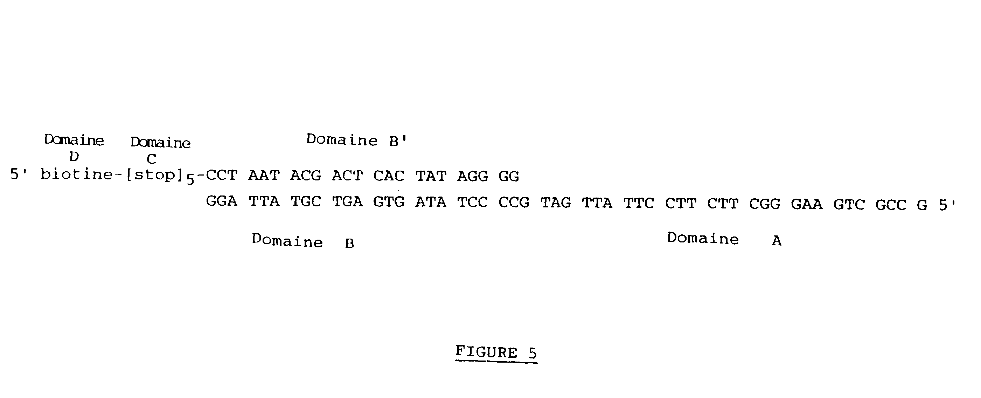

- la figure 5 représente une autre construction polynucléotidique conforme à l'invention.

Il doit être bien entendu, toutefois, que ces exemples sont donnés uniquement à titre d'illustration de l'objet de l'invention, dont ils ne constituent en aucune manière une limitation.

### EXEMPLE 1 : Préparation de la construction.

### a) Synthèse du promoteur (Y) :

- Synthèse du phosphoramidite "stop" :
   6-O-(4,4'-diméthoxytrityl)-hexane-1-O-(2-cyanoéthyl-N,N-diisopropyl)-phosphoramidite.
   L'hexane-1,6-diol (11 g 0,1 mole) est dissous dans le dichlorométhane (150 ml) ; on ajoute de la triéthylamine (22 ml) et de la diméthylaminopyridine (1 g), puis sous agitation (0 à 4°C), on ajoute une solution de 6,8 g (20 mmoles) de chlorure de diméthoxytrityle (DMT) dans 50 ml de dichlorométhane.
   Après 2 heures sous agitation, le mélange est lavé par 200 ml de NaHCO₃ (solution aqueuse à 10 %), puis par 2 X 200 ml d'eau.
   La phase organique est évaporée, puis le composé brut est purifié sur une colonne de silice (silice PF Merck) par chromatographie-flash, en éluant avec un mélange dichlorométhane/hexane (1:1) contenant 1 % de triéthylamine.
   Les fractions renfermant le produit monotritylé de Rf = 0,64 dans le mélange CH₂Cl₂/ET₂O (5:2) sont réunies et évaporées puis co-évaporées au tétrachlorure de carbone. Rendement 3,8 g (51 %).
   Le composé monotritylé obtenu ci-dessus (1,41 g, 3,35 mmoles) est co-évaporé sous-vide par 2 X 20 ml d'acétonitrile anhydre, puis dissous dans 35 ml de dichlorométhane anhydre. On ajoute 285 mg de tétrazolure de diisopropylammonium, puis 4 ml d'une solution de 2-cyanoéthyl-N,N,N',N'-tétraisopropylphosphorodiamidite (1M dans l'acétonitrile anhydre).
   Après 150 min de réaction sous argon et sous agitation, à 20°C, le mélange réactionnel est dilué par 100 ml de dichlorométhane, puis lavé par 2 X 100 ml de NaHCO₃ (solution aqueuse à 10 %) puis par 2 X 100 ml d'une solution de NaCl à demi-saturation.
   La phase organique est concentrée et le résidu est purifié sur une colonne de silice en éluant avec un mélange dichlorométhane/hexane (20/80) contenant 1 % de triéthylamine.
   Après évaporation des solvants, le résidu sirupeux est repris par du benzène anhydre, puis lyophilisé sous-vide. Rendement 1,7 g (80 %).
   Rf = 0,4 dans le mélange Hexane/Et₂O/Et₃N (15/10 /1)
   RMN-1H (CD₃COCD₃): 7,6-7,3 (m, 9H) DMT ; 7,1-6,9 (m, 4H) DMT ; 3,9 (s, 6H) 2 X CH₃O ; 3,8-3,6 (m, 6H) CH₂OP ; CH₂CH₂CN ; 2 X CHipr ; 3,25-3,15 (m, 2H) CH₂ ODMT ; 2,84 (t, 2H) CH₂CN ; 1,8-1,65 (m, 8H) CH₂ ; 1,30 (m, 12H) 4 X CH₃.
   RMN-³¹P (C₆D₆) : 149,3 ; SM(FAB+) : MNa⁺ = 643.
- Promoteur proprement dit : domaine (D)+(C)+(B') :
   La séquence du promoteur (23-mer) est synthétisée sur un synthétiseur d'oligonucléotide Applied Bio-systems, en utilisant une colonne de 1 µmole.
   Le rendement moyen par étape est de 98,2 %. L'oligonucléotide (23-mer) reste tritylé à son extrémité 5'.
   On couple alors 5 fois de suite le phosphoramidite "stop" décrit ci-dessus, en utilisant une solution (0,1M dans l'acétonitrile anhydre) placée sur la voie "X" du synthétiseur (séquence XXXXXT ; DMT "ON").
   Le rendement moyen par étape est de 98,4 % (mesure photométrique à 500 nm).
   Le dérivé biotine phosphoramidite (ROGET A., BAZIN H. et TEOULE R., Nucleic Acids Res., 1989, 17, 7643-7651) est alors couplé à l'extrémité 5' du dérivé de 28-mers obtenu précédemment, en laissant le groupe diméthoxy tritylé en 5' de l'oligodésoxynucléotide.
   L'oligonucléotide Y (29-mers) alors obtenu est alors déprotégé pendant 16 h à 55°C dans l'ammoniaque à 28 % (tube scellé) et correspond au segment dénommé promoteur ci-dessus et comportant, comme domaine (D), de la biotine, comme domaine (C) le bras espaceur [stop]5 tel que préparé ci-dessus et comme domaine (B'), la séquence de 23 mers ci-dessus.
   Après évaporation de l'ammoniaque, on obtient 103 UA₂₆₀ d'oligonucléotide Y brut, qui est repris par 200 µl de TEAB 10 mM (triéthylammonium bicarbonate) et injecté sur une colonne HPLC (RP-18, Merck®).
   On utilise une colonne Lichrosphère Merck 250 X 10 mm RP-18E (10 µm) avec un gradient de 10 % à 32 % d'acétonitrile dans le TEAA 25 mM (triéthylammonium acétate) en 20 minutes, à un débit de 5 ml/min.
   La fraction correspondant au pic principal (19 min<Rt<20,5 min) est collectée, le produit est évaporé à sec, puis repris dans 1 ml d'acide acétique à 80 %. Après 25 min à 20°C, l'acide acétique est évaporé sous-vide.
   Le résidu est repris dans 200 µl de TEAB 10 mM et dessalé sur une colonne NAP5 (Pharmacia®), on obtient ainsi 12UA₂₆₀ de promoteur Y.

### b) Synthèse de la sonde (X) : domaines (B)+(A)

De manière préférée, le domaine (A), constituant une sonde, est obtenu par synthèse chimique classique d'oligonucléotides. Dans ce cas, la technologie des PAC-phosphoramidites (voir Brevet français 2 596 761) peut avantageusement être utilisée, afin, notamment d'assurer une meilleure déprotection de l'oligonucléotide final. Une bonne déprotection est importante, afin d'éviter l'arrêt prématuré de la réaction et limiter les mauvaises incorporations pendant la transcription. (Milligan et al., Methods Enzym,1989, 180, 51-62). Ledit domaine A peut également être obtenu par amplification PCR asymétrique avec des amorces spécifiques de la séquence cible, dont l'une comporte la séquence (B) à son extrémité 5'. Les quantités d'amorces utilisées sont choisies de façon à ce que le simple-brin résultant de l'amplification corresponde à la séquence 3'(B)-(A) 5'. Cette séquence simple-brin est ensuite purifiée après séparation électrophorétique et extraction du gel par des techniques classiques.

De manière plus précise, la sonde (X) est synthétisée sur un synthétiseur Applied Biosystems en utilisant une colonne de 0,2 µmole.

Pour cette synthèse, les phosphoramidites du type PAC (Phenoxyacetic) sont utilisés (SCHULHOF JC., MOLKO D. et TEOULE R., Nucleic Acids Res., 1987, 15, 397-416).

Le dernier groupe diméthoxytrityle en 5' est conservé à l'issue de la synthèse.

L'oligonucléotide est alors déprotégé par l'ammoniaque à 28 % (2,4 ml) pendant 16 h.

On évapore la solution ammoniacale, on reprend le résidu par 200 µl de TEAB ; après dessalage sur une colonne NAP5 (Pharmacia®), on obtient 44UA₂₆₀ d'oligonucléotide brut.

Après concentration, on injecte sur HPLC (colonne Merck® RP-18), colonne RP-18E (5 µm), 125 X 4 mm en éluant avec un gradient de 10 % à 18 % d'acétonitrile en 15 min, puis de 18 % à 50 % d'acétonitrile dans le TEAA 25 mM en 15 min, à un débit de 1 ml/min. On collecte la région centrale du pic (20,6 min<Rt<23,0 min).

On évapore la fraction correspondante et on détrityle (acide acétique 80 %, 20°C, 25 min), puis on évapore à sec sous-vide, on reprend par 200 µl de TEAB 10 mM et on précipite par 2 ml de n-butanol. On obtient ainsi 5 UA₂₆₀ d'oligonucléotide X pur, comprenant le domaine (A), représenté en caractères gras et le domaine (B), représenté en caractères soulignés, suivants :

### c) Hybridation des brins X et Y.

La sonde (X) (4,4 pmoles) et le promoteur (Y) (7,2 pmoles) sont hybridés 15 minutes à 50°C, après 5 min de dénaturation à 99°C, dans 50 µl de tampon SSC 5X, en présence de 10 µg d'ADN de sperme de hareng.

Le duplex ainsi formé peut être stocké et utilisé directement pour l'hybridation avec une cible simple-brin, par exemple, sans étape de dénaturation.

Il peut éventuellement être purifié, afin d'éliminer les promoteurs (Y) non hybridés à la sonde (X).

### EXEMPLE 2 : Résistance du bras espaceur selon l'exemple 1 à l'hydrolyse par les nucléases spécifiques des simples-brins nucléotidiques.

Le promoteur (Y) (3 µl à 0,6 unités de DO) est marqué à son extrémité 5' sur le résidu biotinylé, par la polynucléotide kinase en présence de 3 µl de γ³²P-ATP, dans un volume total de 10 µl. 3 µl de la solution de marquage sont utilisés directement pour tester la résistance de l'oligonucléotide modifié à l'hydrolyse par les nucléases.

Deux enzymes sont testées : la nucléase S1 (Boehringer-Mannheim®, 400 U/µl) et la Mung-Bean-Nucléase (Boehringer-Mannheim®, 50 U/µl).

| * Digestion par la Mung-Bean-Nucléase : | |
|---|---|
| promoteur Y marqué | 3 µl |
| Tampon 5X | 2 µl |
| ADN de sperme de hareng | 2 µl (10 mg/ml) |
| Enzyme | 0,1 µl |
| H₂O | 2,9 µl |
| Incubation 30 minutes à 37°C. | |

| * Digestion par la Nucléase S1 : | |
|---|---|
| promoteur Y marqué | 3 µl |
| Tampon 3X | 3,3 µl |
| ADN de sperme de hareng | 2 µl (10 mg/ml) |
| Enzyme | 0,1 µl |
| H₂O | 1,6 µl |
| Incubation 30 minutes à 37°C. | |

Les réactions de digestion sont arrêtées par addition de 2 µl d'EDTA 0,5M. 5 µl des solutions de digestion sont déposés sur gel d'acrylamide à 25 %, avec le témoin marqué non digéré. Des marqueurs de longueur, marqués au ³²P, sont conjointement déposés sur le gel.

L'autoradiographie montre une disparition totale de l'oligonucléotide initial après digestion par la Mung-Bean-Nuclease et une digestion supérieure à 98 % pour la nucléase S1 ; on observe des résidus très majoritaires (> à 95 %), dans les deux réactions de digestion, dont la taille est d'environ 8 à 9 nucléotides (comparaison avec des marqueurs de longueur). L'oligonucléotide étant marqué à son extrémité 5', ces résidus comprennent le bras espaceur comportant la biotine à son extrémité 5' avec deux ou trois résidus nucléotidiques non hydrolysés à son extrémité 3'.

### EXEMPLE 3 : Test de la capacité de transcription de la construction polynucléotidique selon l'exemple 1, fixée sur un support modifié.

Une construction polynucléotidique telle que préparée à l'exemple 1, en solution, est transférée dans des puits de microplaques recouverts d'avidine. La fixation se déroule dans du tampon SSC 5X, 1 heure à 37°C. Les puits sont lavés dans un tampon PBS + NaCl 0,5 M. La transcription se déroule ensuite en présence de 1 mM de chacun des quatre nucléosides triphophaste, de 0,2 µl de α-³²p-UTP (Amersham®, 800 Ci/mmol, 20 mCi/ml), et de 50 U de T7 ARN polymérase (Gibco-BRL®).

La réaction se déroule 2 heures à 37°C. 7 µl de surnageant de transcription sont prélevés et analysés après migration sur gel d'agarose/Nusieve® (1 %/2 %) et visualisation au bromure d'éthidium ou bien après électrophorèse sur gel d'acrylamide à 25 % et autoradiographie.

L'analyse du gel d'agarose montre une bande très majoritaire de taille identique à celle du domaine A de la sonde (X) dans les puits contenant de la T7 ARN polymérase. Des fragments plus courts minoritaires sont en outre observés. Ces fragments correspondent à des initiations de transcription avortées (Milligan et al., NAR, 1987, 15, 21, 8783-8798). Les puits témoins, où la transcription est effectuée sans enzyme, ne contiennent aucun acide nucléique détectable sur gel. L'analyse des mêmes échantillons sur gel d'acrylamide montre une bande très majoritaire correspondant au transcrit intégral dans les puits contenant l'enzyme de transcription et aucune bande dans les puits témoins ou la transcription est effectuée sans enzyme.

### EXEMPLE 4 : Mise en évidence de la protection de la transcription vis-à-vis de la digestion du domaine (A) de la sonde par des nucléases spécifiques des simple-brins, par hybridation dudit domaine (A) de la sonde avec une séquence complémentaire.

Cet exemple montre la protection de la transcription par hybridation du domaine (A) de la sonde (X) avec une séquence cible complémentaire, lors de l'étape de digestion par les nucléases spécifiques des simple-brins :
5,7 pmoles d'un oligonucléotide synthétique (G) de séquence : sont ajoutés dans le milieu d'hybridation de l'exemple 1.

Cet oligonucléotide a une séquence parfaitement complémentaire du domaine (A) de la sonde. Les espèces présentes sont hybridées et fixées en microplaque comme décrit dans l'exemple 1 et l'exemple 3 ; les puits sont ensuite incubés 30 min à 37°C dans des tampons de digestion contenant la nucléase S1 ou la Mung-Bean-Nuclease et 0,05 mg/ml de BSA.

Après lavages dans un tampon PBS + NaCl 0,5 M + Tween® 20 0,05 %, le milieu de transcription est ajouté dans les puits. La transcription se déroule comme dans l'exemple 3 et les produits sont également analysés comme décrit précédemment.

Après digestion par la Mung-Bean (25 U dans 50 µl) ou par la Nucléase S1 (20 U dans 50 µl), on observe le transcrit de la taille attendue sur gel d'agarose et après autoradiographie.

### EXEMPLE 5 : Effet de la digestion du domaine (A) de la sonde sur l'efficacité de la transcription, en l'absence de séquence complémentaire de (A) pendant la digestion par les nucléases spécifiques des simples-brins : spécificité du test conforme à l'invention.

La sonde (X) et le promoteur (Y) sont hybridés en solution, fixés sur microplaque recouverte d'avidine, digérés et transcrits comme cela est décrit dans les exemples précédents.

L'analyse des produits de transcription par gel d'agarose et par autoradiographie montre l'absence totale, dans les conditions de digestion définies ci-dessus, du transcrit intégral correspondant à la sonde. Le domaine (A) de la sonde non protégé par hybridation est donc digéré pendant l'étape de digestion par les nucléases spécifiques des simples-brins et ceci empêche la production par transcription de l'ARN correspondant au domaine (A) de la sonde.

Ces exemples montrent que le domaine (A) non protégé des nucléases spécifiques des simples-brins est digéré par ces enzymes et que l'ensemble promoteur/sonde digéré n'est plus capable de donner lieu à la transcription du produit attendu. Ils montrent, en outre, que le domaine (A) est protégé de la digestion par les enzymes lorsqu'il est sous la forme d'un double-brin et que l'ensemble construction polynucléotidique/cible donne lieu à une transcription spécifique.

L'exemple 4 montre également que les enzymes testées ne sont pas capables d'induire la coupure de la sonde, entre les domaines (A) et (B) lorsque la cible est parfaitement hybridée sur (A).

### EXEMPLE 6 : Détection d'une séquence cible par le procédé conforme à l'invention (détection du transcrit par hybridation sur membrane).

a) Préparation de la séquence cible :
   La séquence cible est constituée d'un ARN de 150 bases de long, reconnu par le domaine (A) de la construction polynucléotidique selon l'exemple 1 et est obtenue à partir d'un ARNm extrait de tissus d'un patient atteint de leucémie lymphocytaire sévère (ALL, jonction b₂A₂).
   Des dilutions séquentielles de cet ARN sont effectuées (voir résultats ci-après). Le procédé selon l'invention est réalisé sur chacune des dilutions de l'ARN cible.
b) Amplification conforme à l'invention :
   La construction polynucléotidique selon l'invention, comprend la sonde (X) 4,4 pmoles et le promoteur (Y) 7,2 pmoles et les dilutions de la cible sont hybridés en solution comme décrit dans l'exemple 3 en présence de 10 µg d'ADN de sperme de hareng. Après fixation en microplaque, digestion par 16 U de Mung-Bean-Nucléase, en présence de 0,05 mg/ml de BSA, et transcription comme décrit ci-dessus en présence de 1 mg/ml de Tween® 20, les produits de transcription sont analysés comme décrit ci-dessous.
c) Détection de la séquence cible
   10 µl de chacun des surnageants de transcription sont déposés par la technique de dot-blot, sur membrane de Nylon N+ (Amersham®), après dilution dans 90 µl de H₂O et dénaturation 4 min à 90°C. L'ARN est fixé par irradiation UV ; des dépôts de dilutions d'ARN cible initial sont conjointement réalisés dans les mêmes conditions sur la membrane. La membrane est ensuite incubée dans des conditions favorisant l'hybridation spécifique avec une sonde reconnaissant la séquence amplifiée, conformément à l'invention et la séquence cible initiale.

Cette sonde est un oligonucléotide de synthèse (Z) de séquence :

Cet oligonucléotide de synthèse est marqué à son extrémité 5' par du ³²P par réaction de la polynucléotide kinase en présence de γ-³²P-ATP.

Après lavages, les membranes sont autoradio-graphiées et chaque dot est découpé et compté en présence de liquide de scintillation. Les résultats du comptage sont reportés ci-dessous, après soustraction du résultat du blanc (obtenu par réalisation de l'essai lorsque la concentration initiale en ARN cible est nulle).

| * Résultats après mise en oeuvre du procédé selon l'invention : | |
|---|---|
| ARN cible initial | signal mesuré (cpm) |
| 2,3 pmoles | 6475 |
| 0,23 pmoles | 2515 |
| 23 fmoles | 1155 |
| 2,3 fmoles | 611 |
| 0,23 fmoles | 42 |

| * Résultats du témoin non amplifié : | |
|---|---|
| ARN cible initial | signal mesuré (cpm) |
| 7 pmoles | 9471 |
| 0,7 pmoles | 1607 |
| 70 fmoles | 271 |
| 7 fmoles | 76 |
| 0,7 fmoles | 61 |

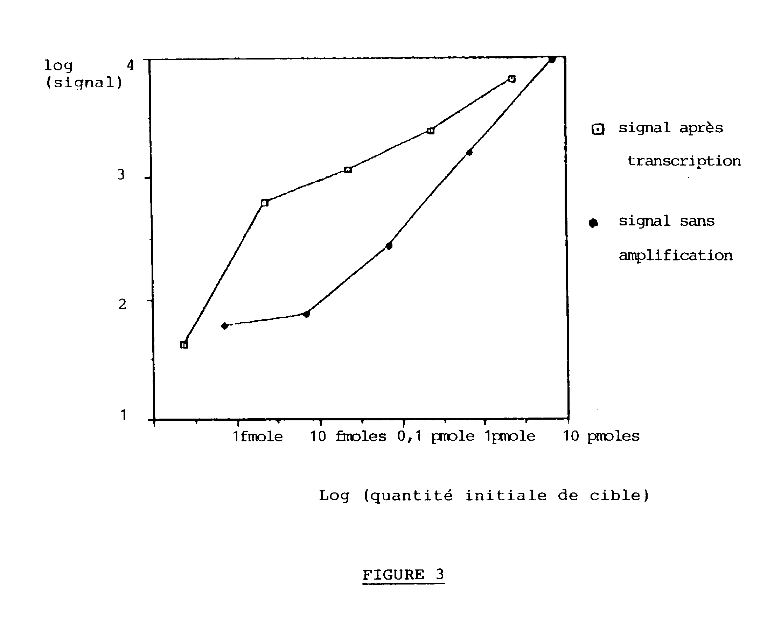

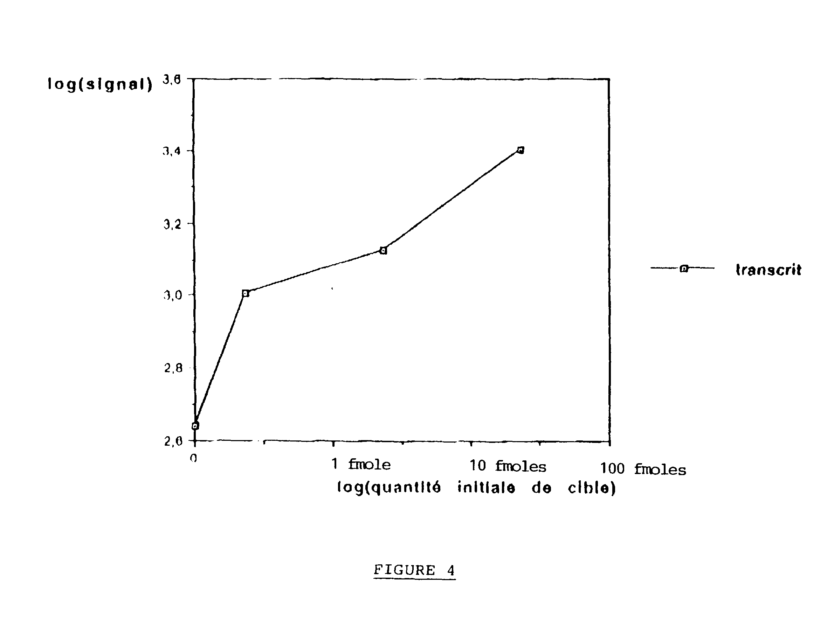

Les résultats peuvent être reportés sur une courbe log(signal) en fonction du log(quantité initiale de cible). Les deux courbes obtenues sont linéaires, dès que le signal, dans les conditions utilisées pour la mesure, est au-dessus du bruit de fond (figure 3 et figure 4). Ces figures montrent que la cible a été détectée de manière sensible et spécifique à l'aide du procédé selon l'invention. Cette amplification est mesurée par la différence de quantité de cible initiale avec amplification et sans amplification donnant un signal identique.

La limite supérieure de cet essai dépend des quantités initiales de construction polynucléotidique présente dans le milieu d'hybridation. L'amplification est limitée lorsque la concentration initiale de la sonde (domaine (A)) est inférieure à la concentration initiale de la cible, elle est par contre plus importante au fur et à mesure que la quantité de cible devient inférieure à la quantité de sonde. Cet effet est expliqué par les lois générales régissant l'hybridation d'une sonde sur sa cible.

Des courbes standards avec des étalons connus peuvent être établies et ainsi permettre la quantification d'un échantillon amplifié dans les mêmes conditions que celles utilisées lors de l'établissement des courbes standards.

De manière préférée, la construction polynucléotidique est à une concentration inférieure à 5 pmoles.

### EXEMPLE 7 : Amplification d'une cible et détection des ARN transcrits, après hybridation en solution avec une sonde spécifique et capture sur phase solide des duplex formés.

Le principe de l'essai est le même que celui décrit dans l'exemple précédent. L'enzyme utilisée est la nucléase S1 (40 U pour 50 µl), la digestion a lieu en présence de 2,5 µg d'ADN de sperme de hareng et 0,05 mg/ml de BSA. La digestion se déroule 20 min à température ambiante. Un marqueur (³²P) est incorporé dans l'ARN transcrit par addition de α-³²P-UTP (0,2 µl par réaction, 800 Ci/mmol, 20 mCi/ml) au milieu de transcription. 10 µl de surnageant de transcription sont prélevés et hybridés en solution en présence de 11 pmoles de sonde (Z) biotinylée à son extrémité 5', dans 30 µl de tampon SSC 2X, contenant 25 mM d'HEPES pH 7,4 et 2 mM d'EDTA. Une hybridation de 15 min à 37°C est réalisée après une étape de dénaturation de 5 min à 70°C. Les solutions sont ensuite transférées dans des puits recouverts d'avidine, contenant 10 µl de SSC 2X. La fixation se déroule 1 heure à 37°C. Les puits sont ensuite lavés avec un tampon PBS + NaCl 0,5 M contenant 0,05 % de Tween® 20. Les cupules sont comptées directement dans un compteur β.

| * Résultats : | |
|---|---|
| ARN cible initial | signal mesuré (cpm) |
| 23 fmoles | 2526 |
| 2,3 fmoles | 1334 |
| 0,23 fmoles | 1011 |
| 0 | 435 |

### EXEMPLE 8 : Test de la capacité de transcription de la sonde/promoteur en solution.

La sonde (X) et le promoteur (Y) sont hybridés comme décrit dans l'exemple 1 c). Les acides nucléiques présents en solution sont ensuite précipités par 5 µl d'acétate de sodium 3M, pH 5,5 avec 100 µl d'éthanol 100 %, 30 minutes à -70°C. Après 15 minutes de centrifugation (10 000 tours/min, 4°C), le culot est lavé par 100 µl d'éthanol 80 % et reprécipité dans les mêmes conditions.

Le culot final est séché et repris par la solution de transcription décrite dans l'exemple 3.

Après 1 heure 30 minutes de transcription à 37°C, 7 µl de la solution sont prélevés et analysés sur gel d'agarose/Nusieve® (1 %/2 %), en présence de bromure d'éthidium. On observe sur le gel :
- une bande correspondant à l'hybride promoteur/sonde ((X)/(Y)) (construction nucléotidique conforme à l'invention),
- une bande correspondant à l'hybride promoteur/sonde/ARN transcrit avec un profil de migration plus lent,
- une bande plus courte correspondant à l'ARN transcrit en solution.

L'autoradiographie du gel montre la présence de radioactivité au niveau de la bande la plus courte (transcrit) et de la bande la plus longue (promoteur/sonde/transcrit).

La même expérimentation réalisée sans T7 ARN polymérase ne permet la visualisation que de la construction conforme à l'invention (promoteur/sonde) sur le gel.

### EXEMPLE 9 : Amplification d'une cible selon le procédé de l'invention et détection colorimétrique des ARN transcrits après immobilisation par une sonde fixée sur microbille.

Le principe de l'essai est le même que celui décrit à l'Exemple 7.

L'enzyme de digestion utilisée est la Mung-Bean nucléase (50 U par essai). La digestion a lieu en présence de 0,05 mg/ml de BSA, pendant 20 min à 37°C.

20 pmoles de sonde (Z) biotinylée en 5', sont fixées sur des microbilles recouvertes d'avidine. La transcription se déroule en présence des billes fonctionnalisées avec la sonde Z, dans un volume final de 50 µl, en présence de ribonucléotides non marqués.

A la fin de la transcription, 20 µl du milieu de transcription contenant les billes sont prélevés et placés dans un tube. On rajoute 20 pmoles (5 µl) de sonde (R) marquée au dinitrophényle à son extrémité 5' (séquence de (R) = 5' AAA CCT TAT TGA TGC 3' (SEQ ID NO.5)) et 25 µl de tampon PBS + NaCl 0,5 M contenant 3 % de BSA. L'hybridation se déroule 1 heure à 37°C.

Les billes sont ensuite récupérées, lavées 3 fois avec 100 µl de tampon PBS + NaCl 0,5 M + Tween® 20 0,05 %. Les transcrits capturés sur billes et hybridés avec la sonde (R) (DNP) sont incubés dans 50 µl de tampon PBS contenant 3 % de BSA et un conjugué anticorps monoclonal anti-DNP couplé à la peroxydase (AMPLICIS-DNP kit ; CIS BIO INTERNATIONAL, dilution 1/50 000). On effectue 3 lavages dans 100 ml de tampon PBS + NaCl 0,5 M + Tween® 0,05 %.

La révélation se déroule dans 50 µl de solution OPD (orthophénylènediamine - DNP kit-CIS BIO INTERNATIONAL) 30 min à l'obscurité. Les valeurs de DO sont mesurées à 492 nm, après addition de 100 µl d'acide oxalique.

Parallèlement, on effectue une hybridation sandwich sur des dilutions de l'ARN cible (10 µl) non soumis au procédé de l'invention, en présence de 20 pmoles (5 µl) de sonde (Z) fixée sur microbilles et 20 pmole (5 µl) de sonde R marquée au DNP, et 25 µl de tampon PBS + NaCl 0,5 M. L'hybridation se déroule 1 heure à 37°C. Les billes sont ensuite lavées et incubées avec de l'anticorps comme décrit ci-dessus.

Résultats (DO 492 nm) :

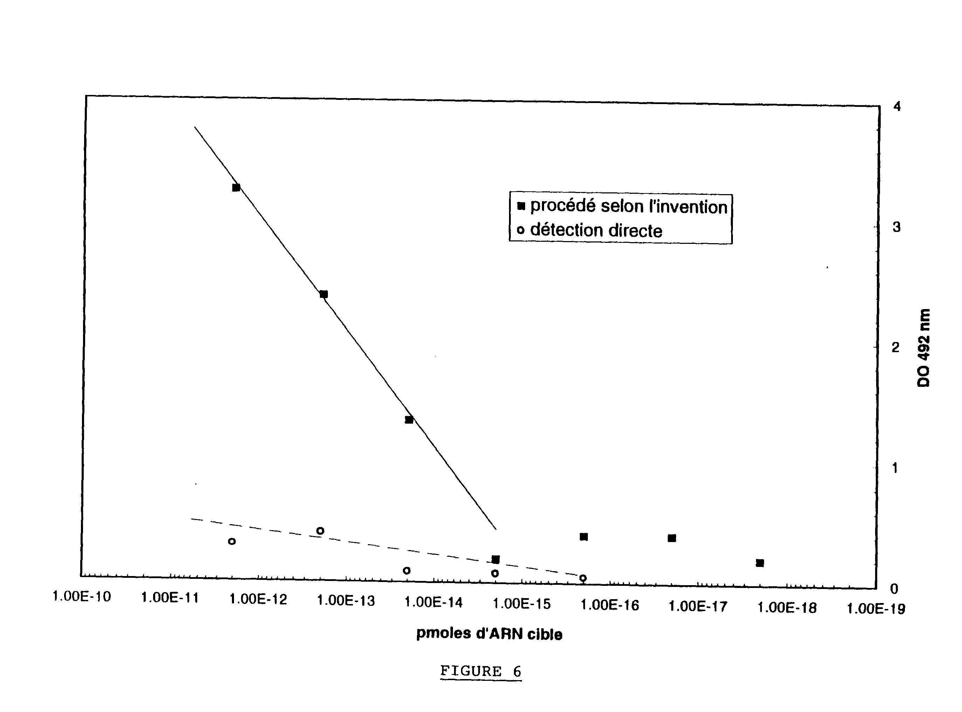

Le Tableau ci-après et la figure 6 (abscisse : pmoles d'ARN cible ; ordonnée : DO à 492 nm) illustrent les résultats (courbe (■) : après amplification selon l'invention ; courbe (O) : par détection directe).

| **ARN cible (pmoles)** | **Après procédé selon l'invention (DO)** | **Sans amplification (DO)** |
|---|---|---|
| 2.10⁻¹² | 3,25 | 0,31 |
| 2.10⁻¹³ | 2,38 | 0,41 |
| 2.10⁻¹⁴ | 1,34 | 0,09 |
| 2.10⁻¹⁵ | 0,2 | 0,08 |
| 2.10⁻¹⁶ | 0,4 | 0,05 |
| 2.10⁻¹⁷ | 0,4 | non testé |
| 2.10⁻¹⁸ | 0,2 | non testé |

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention telle que définie dans les revendications.

## Revendications

1. Procédé d'amplification d'au moins une séquence d'acide nucléique, caractérisé en ce qu'il comprend :
(1) l'hybridation en solution d'au moins une séquence cible avec une construction polynucléotidique comprenant au moins un domaine (A) simple-brin correspondant à une séquence nucléotidique complémentaire de la séquence cible et un domaine (B,B') double-brin constituant un promoteur,
(2) l'élimination des acides nucléiques non hybridés avec le domaine (A) de ladite construction polynucléotidique,
(3) la digestion par une nucléase spécifique des acides nucléiques simple-brin des fragments simple-brin des constructions polynucléotidiques, c'est-à-dire notamment celles n'ayant pas formé d'hybrides avec la séquence cible, et
(4) la transcription en solution et en présence d'une ARN-polymérase ADN-dépendante, de la séquence cible ayant hybridé à l'étape (1), sous la forme de copies multiples d'ARN de ladite séquence cible.

2. Procédé d'amplification selon la revendication 1, caractérisé en ce que l'étape (2) de séparation comprend avantageusement une étape de précipitation des acides nucléiques.

3. Procédé d'amplification selon la revendication 1, caractérisé en ce que la construction polynucléotidique de l'étape (1) comprend au moins un domaine (A) simple-brin correspondant à une séquence nucléotidique complémentaire de la séquence cible, un domaine (B,B') double-brin constituant un promoteur, un domaine (C) constitué par un bras espaceur, non hydrolysable par des nucléases spécifiques des acides nucléiques simple-brin et un domaine (D) constitué par un système de fixation à un support.

4. Procédé d'amplification selon la revendication 3, caractérisé en ce que l'étape (2) de séparation comprend avantageusement une étape de fixation sur un support approprié des constructions polynucléotidiques comportant le domaine (D), suivie d'une étape d'élimination des éléments non fixés.

5. Procédé d'amplification selon la revendication 4, caractérisé en ce que la capacité du support est choisie de telle sorte que tous les composés comportant le domaine (D) puissent se fixer, sans qu'il y ait compétition entre eux.

6. Procédé de détection et de quantification d'au moins une séquence d'acide nucléique, caractérisé en ce qu'il comprend :
(1) l'hybridation en solution d'au moins une séquence cible avec une construction polynucléotidique comprenant au moins un domaine (A) simple-brin correspondant à une séquence nucléotidique complémentaire de la séquence cible et un domaine (B,B') double-brin constituant un promoteur,
(2) l'élimination des acides nucléiques non hybridés avec le domaine (A) de ladite construction polynucléotidique,
(3) la digestion par une nucléase spécifique des acides nucléiques simple-brin des fragments simple-brin des constructions polynucléotidiques, c'est-à-dire notamment celles n'ayant pas formé d'hybrides avec la séquence cible à détecter,
(4) la transcription en solution et en présence d'une ARN-polymérase ADN-dépendante, de la séquence cible ayant hybridé à l'étape (1), sous la forme de copies multiples d'ARN de ladite séquence cible, et
(5) la détection et/ou la quantification des transcrits produits.

7. Procédé de détection selon la revendication 6, caractérisé en ce que l'étape (2) de séparation comprend avantageusement une étape de précipitation des acides nucléiques.

8. Procédé de détection selon la revendication 6, caractérisé en ce que la construction polynucléotidique de l'étape (1) comprend au moins un domaine (A) simple-brin correspondant à une séquence nucléotidique complémentaire de la séquence cible, un domaine (B,B') double-brin constituant un promoteur, un domaine (C) constitué par un bras espaceur, non hydrolysable par des nucléases spécifiques des acides nucléiques simple-brin et un domaine (D) constitué par un système de fixation à un support.

9. Procédé de détection selon la revendication 8, caractérisé en ce que l'étape (2) de séparation comprend avantageusement une étape de fixation sur un support approprié des constructions polynucléotidiques comportant le domaine (D), suivie d'une étape d'élimination des éléments non fixés.

10. Procédé selon l'une quelconque des revendications 1 à 9, caractérisé en ce que l'étape (1) comprend simultanément la formation in situ de la construction polynucléotidique par hybridation d'une séquence comprenant au moins de 5' en 3' le domaine (A) et le domaine (B) avec une séquence comprenant au moins le domaine (B') et de préférence en outre le domaine (D) et le domaine (C) et l'hybridation de ladite construction polynucléotidique ainsi formée et de la séquence cible.

11. Procédé selon l'une quelconque des revendications 1 à 10, caractérisé en ce que l'étape (4) de transcription est réalisée en présence de désoxynucléotides modifiés tels que l'α-³²P-UTP, le dinitrophényl-UTP, la biotine-UTP, la digoxigénine-UTP.

12. Procédé selon l'une quelconque des revendications 6 à 11, caractérisé en ce que lorsque les transcrits sont réalisés en présence de désoxynucléotides modifiés, l'étape (5) de détection comprend la capture desdits transcrits modifiés par une sonde spécifique d'une portion située au centre ou à l'extrémité 3' desdits transcrits et la détection des marqueurs incorporés dans lesdits transcrits par des systèmes de révélation convenables.

13. Procédé selon la revendication 12, caractérisé en ce que la sonde de détection fait avantageusement entre 15 et 200 bases de long et peut être modifiée à une de ses extrémités par une molécule permettant sa fixation sur un support.

14. Procédé selon la revendication 11, caractérisé en ce que l'étape (5) est réalisée en solution, par hybridation du transcrit formé lors de l'étape (4) avec une sonde de détection, dans des conditions favorisant la formation du duplex spécifique et les hybrides ainsi formés sont ensuite immobilisés sur des supports modifiés.

15. Procédé selon l'une quelconque des revendications 6 à 10, caractérisé en ce que l'étape (5) de détection comprend la fixation des transcrits non modifiés sur un support solide et leur hybridation avec une sonde de détection spécifique marquée.

16. Construction polynucléotidique apte à amplifier et/ou éventuellement à détecter une séquence cible d'acide nucléique, caractérisée en ce qu'elle comprend :
(a) un premier domaine (A), qui est simple-brin et comprend une séquence nucléotidique complémentaire de la séquence cible,
(b) un deuxième domaine (B,B'), situé à l'extrémité 3' dudit domaine (A), qui est formé de deux séquences complémentaires (B) et (B') et forme un promoteur double-brin d'une ARN polymérase ADN-dépendante,
(c) un troisième domaine (C), constitué par un bras espaceur, non hydrolysable par des nucléases spécifiques des acides nucléiques simple-brin, et
(d) un quatrième domaine (D), constitué par un système de fixation du domaine (B,B') sur un support solide convenable,
lesquels domaines (C) et (D) peuvent être avantageusement fixés soit en 5' du domaine (B'), soit en 5' du domaine (A), soit en 3' du domaine (B) et forment un système d'accrochage.

17. Construction polynucléotidique selon la revendication 16, caractérisée en ce que le domaine (B,B') constitue l'un des promoteurs suivants : promoteur de la T7 ARN polymérase, promoteur de la T3 ARN polymérase, promoteur de la SP6 ARN polymérase.

18. Construction polynucléotidique selon la revendication 16 ou la revendication 17, caractérisée en ce que des séquences peuvent être ajoutées à l'extrémité 3' et/ou à l'extrémité 5' du domaine (B).

19. Construction polynucléotidique selon l'une quelconque des revendications 16 à 18, caractérisée en ce que la taille de la séquence formée par les domaines (A) et (B) est comprise entre 30 et 200 bases.

20. Construction polynucléotidique selon l'une quelconque des revendications 16 à 19, caractérisée en ce que le domaine (C) ou bras espaceur est avantageusement constitué de 2 à 10, de préférence de 4 à 6 synthons, choisis parmi les alkanes-diol ou les synthons qui répondent à l'une des formules suivantes :
-R₁- [(CH₂)ₙ-R₂]ₓ- [(CH₂)ₘ-R₃]_{y}- (CH₂)ₚ-
dans laquelle :
n est un nombre entier de 1 à 10 ;
m est égal à 0 ou est un nombre entier de 1 à 10 ;
p est égal à 0 ou est un nombre entier de 1 à 10 ;
x est égal à 0 ou est un nombre entier de 1 à 8 ;
y est égal à 0 ou est un nombre entier de 1 à 8 ;
R₁, R₂ et R₃, qui peuvent être identiques ou différents représentent CH₂ ; O ; S ; NR' ; CO ; CH=CH ; NR'CO ; CONR' ; NHSO₂ ; R'PO₄,
où R' représente un atome d'hydrogène ou une chaîne alkyle en C₁ à C₁₂.

21. Construction polynucléotidique selon l'une quelconque des revendications 16 à 20, caractérisée en ce que le domaine (D) est avantageusement un oligonucléotide, un haptène, du dinitrophényle, une biotine, ou une digoxigénine.

22. Procédé de production d'une construction polynucléotidique selon l'une quelconque des revendications 16 à 21, caractérisé en ce qu'il comprend :
(a) la synthèse du segment dénommé promoteur comprenant le domaine (D), le domaine (C) (bras espaceur) et le domaine 5'-(B')-3' (correspondant à un simple-brin d'un promoteur),
(b) la synthèse du segment dénommé sonde, comprenant le domaine 3'-(B) (correspondant à une séquence simple-brin complémentaire de (B')), le domaine (A)-5' (séquence simple-brin complémentaire de la séquence cible à détecter) et
(c) l'hybridation des domaines (B) et (B'), des segments obtenus en (a) et (b), dans des conditions stringentes.

23. Procédé de production d'une construction polynucléotidique selon l'une quelconque des revendications 16 à 21, caractérisé en ce qu'il comprend :
(a) la synthèse du segment dénommé promoteur comprenant uniquement le domaine 5'-(B')-3' (correspondant à un simple-brin d'un promoteur),
(b) la synthèse du segment dénommé sonde, comprenant le domaine 3'-(B) (correspondant à une séquence simple-brin complémentaire de (B')), le domaine (A)-5' (séquence simple-brin complémentaire de la séquence cible à détecter) et en 3'ou en 5' dudit segment, le domaine (C) (bras espaceur) et le domaine (D) (système d'accrochage), et
(c) l'hybridation des domaines (B) et (B'), des segments obtenus en (a) et (b), dans des conditions stringentes.

## Patentansprüche

1. Verfahren zur Amplifikation mindestens einer Nucleinsäuresequenz, dadurch gekennzeichnet, daß man
(1) in Lösunq mindestens eine Zielsequenz mit einer Polynucleotidkonstruktion, die mindestens eine einzelsträngige Domäne (A), die einer der Zielsequenz komplementären Nucleotidsequenz entspricht, und eine doppelsträngige Domäne (B,B'), die einen Promotor bildet, umfaßt, hybridisiert,
(2) mit der Domäne (A) der Polynucleotidkonstruktion nicht hybridisierte Nucleinsäuren entfernt,
(3) mit einer für einzelsträngige Nucleinsäuren spezifischen Nuclease einzelsträngige Fragmente der Polynucleotidkonstruktion, d.h. insbesondere diejenigen, die mit der Zielsequenz keine Hybride gebildet haben, spaltet, und
(4) in Lösung und in Anwesenheit einer DNA-abhängigen RNA-Polymerase die Zielsequenz, die in Stufe (1) hybridisiert hat, in Form von Mehrfachkopien der RNA der Zielsequenz transkribiert.

2. Verfahren zur Amplifikation nach Anspruch 1, dadurch gekennzeichnet, daß die Stufe (2) der Abtrennung vorteilhafterweise eine Stufe der Fällung der Nucleinsäuren umfaßt.

3. Verfahren zur Amplifikation nach Anspruch 1, dadurch gekennzeichnet, daß die Polynucleotidkonstruktion der Stufe (1) mindestens eine einzelsträngige Domäne (A), die einer der Zielsequenz komplementären Nucleotidsequenz entspricht, eine doppelsträngige Domäne (B,B'), die einen Promotor darstellt, eine aus einem Spacer-Arm bestehende Domäne (C), die durch Nucleasen, die für die einzelsträngigen Nucleinsäuren spezifisch sind, nicht hydrolysierbar ist, und eine Domäne (D), die aus einem System zur Fixierung an einen Träger besteht, umfaßt.

4. Verfahren zur Amplifikation nach Anspruch 3, dadurch gekennzeichnet, daß die Stufe (2) der Abtrennung vorteilhafterweise eine Stufe der Fixierung der die Domäne (D) umfassenden Polynucleotidkonstruktionen an einem geeigneten Träger umfaßt, gefolgt von einer Stufe der Entfernung der nicht fixierten Bestandteile.

5. Verfahren zur Amplifikation nach Anspruch 4, dadurch gekennzeichnet, daß man die Kapazität des Trägers so wählt, daß alle Verbindungen, die die Domäne (D) enthalten, ohne Kompetition untereinander gebunden werden können.

6. Verfahren zum Nachweis und zur quantitativen Bestimmung mindestens einer Nucleinsäuresequenz, dadurch gekennzeichnet, daß man
(1) in Lösung mindestens eine Zielsequenz mit einer Polynucleotidkonstruktion, die mindestens eine einzelsträngige Domäne (A), die einer der Zielsequenz komplementären Nucleotidsequenz entspricht, und eine doppelsträngige Domäne (B,B'), die einen Promotor bildet, umfaßt, hybridisiert,
(2) mit der Domäne (A) der Polynucleotidkonstruktion nicht hybridisierte Nucleinsäuren entfernt,
(3) mit einer für einzelsträngige Nucleinsäuren spezifischen Nuclease einzelsträngige Fragmente der Polynucleotidkonstruktionen abspaltet, d.h. insbesondere diejenigen, die mit der nachzuweisenden Zielsequenz keine Hybride gebildet haben,
(4) in Lösung und in Anwesenheit einer DNA-abhängigen RNA-Polymerase die Zielsequenz, die in Stufe (1) hybridisiert hat, in Form von Mehrfachkopien der RNA der Zielsequenz transkribiert und
(5) die transkribierten Produkte nachweist und/oder quantitativ bestimmt.

7. Verfahren zum Nachweis nach Anspruch 6, dadurch gekennzeichnet, daß die Stufe (2) der Abtrennung vorteilhafterweise eine Stufe der Fällung der Nucleinsäuren umfaßt.

8. Verfahren zum Nachweis nach Anspruch 6, dadurch gekennzeichnet, daß die Polynucleotidkonstruktion der Stufe (1) mindestens eine einzeisträngige Domäne (A), die einer der Zielsequenz komplementären Nucleotidsequenz entspricht, eine doppelsträngige Domäne (B,B'), die einen Promotor bildet, eine Domäne (C), die aus einem Spacer-Arm besteht, der durch für einzelsträngige Nucleinsäuren spezifische Nucleasen nicht hydrolysierbar ist, und eine Domäne (D), die aus einem System zur Fixierung an einen Träger besteht, umfaßt.

9. Verfahren zum Nachweis nach Anspruch 8, dadurch gekennzeichnet, daß die Stufe (2) der Abtrennung vorteilhafterweise eine Stufe der Fixierung der Polynucleotidkonstruktionen, die die Domäne (D) enhalten, an einen geeigneten Träger umfaßt, gefolgt von einer Stufe der Entfernung der nicht gebundenen Bestandteile.

10. Verfahren nach einem der Ansprüche 1 bis 9, dadurch gekennzeichnet, daß die Stufe (1) gleichzeitig die in situ-Bildung der Polynucleotidkonstruktion durch Hybridisierung einer Sequenz, die in 5'-3'-Richtung mindestens die Domäne (A) und die Domäne (B) umfaßt, mit einer Sequenz, die mindestens die Domäne (B') und bevorzugt weiterhin die Domäne (D) und die Domäne (C) umfaßt, und die Hybridisierung der so gebildeten Polynucleotidkonstruktion und der Zielsequenz umfaßt.

11. Verfahren nach einem der Ansprüche 1 bis 10, dadurch gekennzeichnet, daß man die Stufe (4) der Transkription in Gegenwart von modifizierten Desoxynucleotiden wie α-^{32p}-UTP, Dinitrophenyl-UTP, Biotin-UTP, Digoxygenin-UTP durchführt.

12. Verfahren nach einem der Ansprüche 6 bis 11, dadurch gekennzeichnet, daß, wenn die Transkripte in Gegenwart von modifizierten Desoxynucleoiiden erzeugt werden, die Stufe (5) des Nachweises das Einfangen der Transkripte, die mit einer Sonde, die für einen Teil, der in der Mitte oder am 3'-Ende der Transkripte lokalisiert ist, spezifisch ist, modifiziert sind, und den Nachweis der in die Transkripte eingebauten Marker mit geeigneten Detektionssystemen umfaßt.

13. Verfahren nach Anspruch 12, dadurch gekennzeichnet, daß die Detektionssonde vorteilhafterweise 15 bis 200 Basen lang ist und an einem ihrer Enden durch ein Molekül modifiziert sein kann, das dessen Fixierung an einem Träger erlaubt.

14. Verfahren nach Anspruch 11, dadurch gekennzeichet, daß die Stufe (5) in Lösung durch Hybridisierung des während der Stufe (4) gebildeten Transkripts mit einer Nachweissonde unter Bedingungen, die für die Bildung eines spezifischen Doppelstrangs günstig sind, durchgeführt wird,und die so gebildeten Hybride anschließend auf den modifizierten Trägern immobilisiert werden.

15. Verfahren nach einem der Ansprüche 6 bis 10, dadurch gekennzeichnet, daß die Stufe (5) des Nachweises die Fixierung der nicht modifizierten Transkripte an einem festen Träger und ihre Hybridisierung mit einer spezifischen, markierten Detektionssonde umfaßt.

16. Polynucleotidkonstruktion mit der Eignung zur Amplifikation und/oder ggf. zum Nachweis einer Nucleinsäurezielsequenz, dadurch gekennzeichnet, daß sie umfaßt
(a) eine erste Domäne (A), die einzelsträngig ist und eine der Zielsequenz komplementäre Nucleotidsequenz umfaßt,
(b) eine zweite Domäne (B,B'),die am 3'-Ende der Domäne (A) lokalisiert ist, die aus zwei komplementären Sequenzen (B) und (B') gebildet ist und einen doppelsträngigen Promotor einer DNA-abhängigen RNA-Polymerase bildet,
(c) eine dritte Domäne (C), die aus einem durch für einzelsträngige Nucleinsäuren spezifische Nucleasen nicht hydrolysierbaren Spacer-Arm besteht, und
(d) eine vierte Domäne (D), die aus einem System zur Fixierung der Domäne (B,B') an einem geeigneten, festen Träger besteht, wobei die Domänen (C) und (D) vorteilhafterweise entweder 5' der Domäne (B') oder 5' der Domäne (A) oder 3' der Domäne (B) gebunden sein können, und ein Anheftungssystem bilden.

17. Polynucleotidkonstruktion nach Anspruch 16, dadurch gekennzeichnet, daß die Domäne (B,B') einen der folgenden Promotoren bedeutet: Promotor der T7-RNA-Polymerase, Promotor der T3-RNA-Polymerase, Promotor der SP6-RNA-Polymerase.

18. Polynucleotidkonstruktion nach Anspruch 16 oder 17, dadurch gekennzeichnet,daß die Sequenzen am 3'-Ende und/oder am 5'-Ende der Domäne (B) angefügt sein können.

19. Polynucleotidkonstruktion nach einem der Ansprüche 16 bis 18, dadurch gekennzeichnet, daß die Größe der durch die Domänen (A) und(B) gebildeten Sequenz zwischen 30 und 200 Basen liegt.

20. Polynucleotidkonstruktion nach einem der Ansprüche 16 bis 19, dadurch gekennzeichnet, daß die Domäne (C) oder der Spacer-Arm vorteilhafterweise aus 2 bis 10, bevorzugt 4 bis 6 Synthons, ausgewählt aus Alkandiolen, oder den Synthons entsprechend einer der folgenden Formeln besteht:
-R₁-[(CH₂)ₙ-R₂]ₓ-[(CH₂)ₘ-R₃]_{y}-(CH₂)ₚ -
worin:
n eine ganze Zahl von 1 bis 10 ist;
m 0 oder eine ganze Zahl von 1 bis 10 ist;
p 0 oder eine ganze Zahl von 1 bis 10 ist;
x 0 oder eine ganze Zahl von 1 bis 8 ist;
y 0 oder eine ganze Zahl von 1 bis 8 ist;
R₁, R₂ und R₃, die gleich oder verschieden sein können, CH₂; O, S; NR'; CO; CH=CH; NR'CO; CONR'; NHSO₂; R'PO₄ bedeuten und R' ein Wasserstoffatom oder eine C₁-C₁₂-Alkylkette bedeutet.

21. Polynucleotidkonstruktion nach einem der Ansprüche 16 bis 20, dadurch gekennzeichnet, daß die Domäne (D) vorteilhafterweise ein Oligonucleotid, ein Hapten, Dinitrophenyl, ein Biotin oder ein Digoxigenin ist.

22. Verfahren zur Herstellung einer Polynucleotidkonstruktion nach einem der Ansprüche 16 bis 21, dadurch gekennzeichnet, daß man
(a) das als Promotor bezeichnete Segment, umfassend die Domäne (D), die Domäne (C) (Spacer-Arm) und die Domäne 5'-(B')-3' (entsprechend einem Einzelstrang des Promotors), synthetisiert,
(b) das als Sonde bezeichnete Segment, umfassend die Domäne 3'-B (entsprechend einer einzelsträngigen Sequenz, die B' komplementär ist), die Domäne (A)-5' (einzelsträngige Sequenz, die der nachzuweisenden Zielsequenz komplementär ist) synthetisiert, und
(c) die Domänen (B) und (B'), die in (a) und (b) enthaltenen Segmente, unter stringenten Bedingungen hybridisiert.

23. Verfahren zur Herstellung einer Polynucleotidkonstruktion nach einem der Ansprüche 16 bis 21, dadurch gekennzeichnet, daß man
(a) das als Promotor bezeichnete Segment, umfassend ausschließlich die Domäne 5'-(B')-3' (entsprechend einem Einzelstrang eines Promotors), synthetisiert,
(b) das als Sonde bezeichnete Segment, umfassend die Domäne 3'-(B) (entsprechend einer einzelsträngigen Sequenz, die (B') komplementär ist), die Domäne (A)-5' (einzelsträngige Sequenz, die der nachzuweisenden Zielsequenz komplementär ist) und 3' oder 5' des Segments, die Domäne (C) (Spacer-Arm) und die Domäne (D) (Anheftungssystem), synthetisiert und
(c) die Domänen (B) und (B') der in (a) und (b) erhaltenen Segmente unter stringenten Bedingungen hybridisiert.

## Claims

1. A process for the amplification of at least one nucleic acid sequence, characterised in that it comprises:
(1) hybridisation in solution of at least one target sequence with a polynucleotidic construction comprising at least one single-strand area (A) corresponding to a nucleotide sequence which is complementary to the target sequence and a double-strand area (B, B') constituting a promoter,
(2) elimination of the non-hybridised nucleic acids with the stage (A) of the said polynucleotide construction,
(3) digestion by a specific nuclease of the single-strand nucleic acids of the single-strand fragments ofthe polynucleotide construction, that is to say particularly those which have not formed hybrids with the target sequence, and
(4) transcription in solution and in the presence of a DNA-dependent RNA polymerase of the target sequence which has hybridised at stage (1) in the form of multiple copies ofRNA of the said target sequence.

2. An amplification process according to claim 1, characterised in that the separation stage (2) advantageously comprises a stage involving precipitation of the nucleic acids.

3. An amplification process according to claim 1, characterised in that the polynucleotide construction of stage (1) comprises at least one single-strand area (A) corresponding to a nucleotide sequence complementary to the target sequence, a double-strand area (B, B') constituting a promoter, an area (C) constituted by a spacer arm which cannot be hydrolysed by specific nucleases of single-strand nucleic acids and an area (D) constituted by a system of fixing on a carrier.

4. An amplification process according to claim 3, characterised in that the separation stage (2) advantageously comprises a stage of fixing on a suitable carrier of polynucleotide constructions comprising the area (D) followed by a stage resulting in elimination of elements which are not fixed.

5. An amplification process according to claim 4, characterised in that the carrier capacity is so chosen that all the compounds comprising area (D) can be fixed without there being any competition among them.

6. A process for the detection and qualification of at least one nucleic acid sequence, characterised in that it comprises:
(1) hybridisation in solution of at least one target sequence with a polynucleotide construction comprising at least one single-strand area (A) corresponding to a nucleotide sequence complementary to the target sequence and a double-strand area (B, B') constituting a promoter;
(2) elimination of non-hybridised nucleic acids with the area (A) of the said polynucleotide construction;
(3) digestion by specific nuclease of the single-strand nucleic acids of single-strand fragments of polynucleotide construction, that is to particularly those which have not formed hybrids with the target sequence to be detected;
(4) transcription in solution and in the presence of a DNA-dependent RNA polymerase of the target sequence which has hybridised in stage (1), in the form of multiple copies ofRNA of the said target sequence, and
(5) the detection and/or quantification of the products transcribed.

7. A detection process according to claim 6, characterised in that the separation stage (2) advantageously comprises a nucleic acid precipitation stage.

8. A detection process according to claim 6, characterised in that the polynucleotide construction in stage (1) comprises at least one single-strand area (A) corresponding to a nucleotide sequence which is complementary to the target sequence, a double-strand area (B, B') constituting a promoter, an area (C) constituted by a spacer arm which cannot be hydrolysed by specific nucleases of single-strand nucleic acids and an area (D) constituted by a system of fixing on a carrier.

9. A detection process according to claim 8, characterised in that the separation stage (2) advantageously comprises a stage involving fixing on a suitable carrier of polynucleotide constructions comprising the area (D) followed by a stage of elimination of the elements which are not fixed.

10. A process according to any one of claims 1 to 9, characterised in that stage (1) simultaneously comprises the *in situ* formation of the polynucleotide construction by hybridisation of a sequence comprising at least from 5' to 3' the area (A) on the area (B) with a sequence comprising at least the area (B') and preferably also the area (D) and the area (C) and hybridisation of the said polynucleotide construction which is thus formed and of the target sequence.

11. A process according to any one of claims 1 to 10, characterised in that the transcription stage (4) is carried out in the presence of modified deoxynucleotides such as α-³²p-UTP, dinitrophenyl-UTP, biotin-UTP, digoxigenin-UTP.

12. A process according to any one of claims 6 to 11, characterised in that when the transcripts are formed in the presence of modified deoxynucleotides, the detection stage (5) comprises the capture of the said modified transcripts by a specific probe of a portion situated at the centre or at the end 3' of the said transcripts and detection of markers incorporated in the said transcripts by suitable revelation systems.

13. A process according to claim 12, characterised in that the detection probe advantageously makes between 15 and 200 bases long and may be modified at one of its ends by a molecule by which it can be fixed on a carrier.

14. A process according to claim 11, characterised in that stage (5) is carried out in solution by hybridisation of the transcript formed during stage (4) with a detection probe, under conditions which favour the formation of the specific duplex and the hybrids thus formed are then immobilised on modified carriers.

15. A process according to any one of claims 6 to 10, characterised in that the detection stage (5) is fixing of the non-modified transcripts on a solid carrier and their hybridisation with a specific marked detection probe.

16. A polynucleotide construction adapted to amplify and/or possibly detect a target sequence of nucleic acid characterised in that it comprises:
(a) a first area (A) which is single-strand and comprises a nucleotide sequence complementary to the target sequence;
(b) a second area (B, B') situated at the end 3' of the said area (A) and which is formed by two complementary sequences (B) and (B') and forms a double-strand promoter of a DNA-dependent RNA polymerase,
(c) a third area (C) constituted by a spacer arm which cannot be hydrolysed by specific nucleases of single-strand nucleic acids, and
(d) a fourth area (D) constituted by a system for fixing the area (B, B') on a suitable solid carrier,
which areas (C) and (D) may advantageously be fixed either at 5' of area (B') or at 5' of area (A) or at 3' of area (B) and form an engaging system.

17. A polynucleotide construction according to claim 16, characterised in that the area (B, B') constitutes one of the following promoters: promoter of T7 RNA polymerase, promoter of T3 RNA polymerase, promoter of SP6 RNA polymerase.

18. A polynucleotide construction according to claim 16 or claim 17, characterised in that sequences may be added to the end 3' and/or to the end 5' of the area (B).

19. A polynucleotide construction according to any one of claims 16 to 18, characterised in that the size of the sequence formed by areas (a) and (B) is comprised between 30 and 200 bases.

20. A polynucleotide construction according to any one of claims 16 to 19, characterised in that the area (C) or a spacer arm is advantageously constituted by 2 to 10 and preferably 4 to 6 synthons chosen from among the alkanes diol or the synthons which satisfy one of the following formulae:
-R₁-[(CH₂)ₙ-R₂]ₓ-[(CH₂)ₘ-R3]_{y}-CH₂)ₚ-
in which
n is a whole number from 1 to 10;
m is equal to 0 or is a whole number from 1 to 10;
p is equal to 0 or is a whole number from 1 to 10;
x is equal to 0 or is a whole number from 1 to 8;
y is equal to 0 or is a whole number from 1 to 8;
R_{1,} R₂ and R₃ which may be identical or different represent
CH₂; O; S; NR'; CO; CH=CH; NR'CO'; CONR'; NHSO₂; R'PO₄ in which R' represents a hydrogen atom or an alkyl chain at C₁ to C₁₂.

21. A polynucleotide construction according to any one of claims 16 to 20, characterised in that the area (D) is advantageously an oligonucleotide, a haptene, dinitrophenyl, a biotin or a digoxigenin.

22. A method of producing a polynucleotide construction according to any one of claims 16 to 21, characterised in that it comprises:
(a) synthesis of the segment styled as a promoter and comprising the area (D), the area (C) (spacer arm) and the area 5'-(B')-3' (corresponding to a single-strand of a promoter);
(b) synthesis of the segment designated probe comprising the area 3'-(B), (corresponding to a single-strand sequence complementary to (B'-)), the area (A)-5' (single-strand sequence complementary to the target sequence to be detected), and
(c) hybridisation of the areas (B) and (B') of the segments obtained in (A) and (B) under strict condition s.

23. A method of producing a polynucleotide construction according to any one of claims 6 to 21, characterised in that it comprises:
(a) synthesis of the segment referred to a promoter comprising only the area 5'-(B')-3' (corresponding to a single-strand of a promoter);
(b) synthesis of the segment referred to as probe comprising the area 3'-(B) (corresponding to a single-strand sequence complementary to (B')), the area (A)-5' (single-strand sequence complementary to the target sequence to be detected) and at 3' or at 5' of the said segment, the area (C) (spacer arm) and the area (D) (attachment system),
(c) hybridisation of the areas (B) and (B') of the segments obtained in (a) and (b) under strict conditions.


### LISTE DE SEQUENCES

(1) INFORMATIONS GENERALES:
   (i) DEPOSANT:
      (A) NOM: CIS BIO INTERNATIONAL
      (B) RUE: RN 306
      (C) VILLE: SACLAY
      (E) PAYS: FRANCE
      (F) CODE POSTAL: 91400
   (ii) TITRE DE L' INVENTION: METHODE D'AMPLIFICATION ET/OU DE DETECTION D'UNE SEQUENCE D'ACIDE NUCLEIQUE, REACTIF DE DETECTION ET LEURS APPLICATIONS.
   (iii) NOMBRE DE SEQUENCES: 5
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "SONDE SYNTHETIQUE"
   (xi) DESCRIPTION DE LA SEQUENCE: SEQ ID NO: 1:
   CCTAATACGA CTCACTATAG GGG                                    23
(2) INFORMATIONS POUR LA SEQ ID NO: 2:
   (i) CARACTERISTIQUES DE LA SEQUENCE:
      (A) LONGUEUR: 52 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "SONDE SYNTHETIQUE"
   (xi) DESCRIPTION DE LA SEQUENCE: SEQ ID NO: 2:
   GCCGCTGAAG GGCTTCTTCC TTATTGATGC CCCTATAGTG AGTCGTATTA GG 52
(2) INFORMATIONS POUR LA SEQ ID NO: 3:
   (i) CARACTERISTIQUES DE LA SEQUENCE:
      (A) LONGUEUR: 29 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "SONDE SYNTHETIQUE"
   (xi) DESCRIPTION DE LA SEQUENCE: SEQ ID NO: 3:
   CATCAATAAG GAAGAAGCCC TTCAGCGGC 29
(2) INFORMATIONS POUR LA SEQ ID NO: 4:
   (i) CARACTERISTIQUES DE LA SEQUENCE:
      (A) LONGUEUR: 15 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "SONDE SYNTHETIQUE"
   (xi) DESCRIPTION DE LA SEQUENCE: SEQ ID NO: 4:
   GCCGCTGAAG GGCTT 15
(2) INFORMATIONS POUR LA SEQ ID NO: 5:
   (i) CARACTERISTIQUES DE LA SEQUENCE:
      (A) LONGUEUR: 15 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "SONDE SYNTHETIQUE"
   (xi) DESCRIPTION DE LA SEQUENCE: SEQ ID NO: 5:
   AAACCTTATT GATGC 15